# EUROPÄISCHE PATENTANMELDUNG

(11) **EP 0 578 943 A2**
(43) Veröffentlichungstag der Anmeldung: **19.01.1994**
(21) Anmeldenummer: 93107622.8
(22) Anmeldetag: 11.05.1993
(51) Int. Cl.: C07C 2/70, C07C 15/02, C07C 15/107

(54) **Verfahren zur Herstellung linearer Alkylbenzole mit besonders niedriger Bromzahl**

(30) Priorität: 15.07.1992 DE 4223206
(71) Anmelder: HÜLS AKTIENGESELLSCHAFT, D-45764 Marl (DE)
(72) Erfinder: Hons, Gerd, Dr., W-4690 Herne 2 (DE); Hosemann, Helmut, W-4270 Dorsten (DE); Kosswig, Kurt, Dr., W-4370 Marl (DE); Schulze, Klaus, Dr., W-4358 Haltern (DE); Wienhöfer, Ekkehard, Dr., W-4370 Marl (DE)

(57) **Zusammenfassung**

Die Erfindung betrifft ein zweistufiges Verfahren zur Herstellung von linearen Alkylbenzolen mit Bromzahlen niedriger als 0,003 [g Br₂/100 g LAB] als Vorprodukt für Detergenzien mit verbesserter Farbzahl durch Umsetzung von überschüssigem Benzol mit einem Olefinschnitt mit einem Monoolefingehalt von mindestens 95 % unter Verwendung von reinem Fluorwasserstoff als Katalysator in einer ersten Stufe und Raffination des destillierten linearen Alkylbenzols mit Frischbenzol und redestilliertem Fluorwasserstoff in einer zweiten Mischstufe mit intensiver Durchmischung des Fluorwasserstoffs mit der organischen Phase.

## Beschreibung

Die Erfindung betrifft ein Verfahren zur Herstellung von besonders reinen linearen Alkylbenzolen mittels Flußsäure katalysierter Alkylierung von Benzol mit Olefinen in zwei Stufen, wobei in der ersten Stufe ein Olefinschnitt mit besonders hohem Monoolefingehalt eingesetzt wird, an den sich als zweite Stufe ein besonderer Raffinationsschritt mit einer intensiven Durchmischung in einer geeigneten Mischeinrichtung und nachfolgender Aufarbeitung mit Rückführung reinen Fluorwasserstoffs anschließt.

Die Alkylierung aromatischer Kohlenwasserstoffe mit unverzweigten Monolefinen mit innenstehender Doppelbindung im Gemisch mit Paraffinen unter Verwendung von flüssigem Fluorwasserstoff (HF) als Katalysator ist seit langem bekannt und wird technisch zur Herstellung großer Mengen linearer Alkylbenzole (im folgenden LAB genannt), die als Rohstoffe für Detergenzien eingesetzt werden, angewendet. Der entsprechende Stand der Technik ist beispielsweise in US-PS 3 950 448, in dem die Verwendung von zwei in Serie geschalteten Alkylierungsreaktoren und Absitzern beschrieben wird, zu entnehmen. Dort wird auch eine Methode zur Regeneration des Fluorwasserstoffs beschrieben. US-PS 3 494 971 beinhaltet eine Variante des Einsatzes zweier Alkylierungsreaktoren. Weiter ist die Herstellung von LAB's in den US-PSS 3 275 702; 3 501 543; 3 501 544; 3 830 865; 3 484 498 beschrieben.

In den US-PSS 4 467 128 und 4 503 277 werden Verfahren zur Regeneration von HF im Alkylierungsprozess beschrieben.

Alle o.g. Erfindungen sind geeignet, die Herstellkosten zu senken, weil der HF-Verbrauch und/oder die Bildung von Nebenprodukten, wie z. B. Säureteer, reduziert, die LAB-Ausbeute erhöht und im Aufarbeitungsteil auf Kolonnen verzichtet werden kann. Alle bisherigen Verfahren führen zu einem LAB herkömmlicher Qualität, die gekennzeichnet ist durch einen Gehalt an Dialkyltetralinen und -indanen ≧ 0,3 % und Bromzahlen von ≧ 0,006 [g Br₂/100 g LAB]. Diese LAB's führen bei der üblichen Weiterverarbeitung zu Säuren mit Jodfarbzahlen ≧ 1 und deutlich gefärbten Konzentraten, was in verbrauchernahen Detergenzien unerwünscht ist.

Eine wirklich entscheidende Verbesserung der LAB-Qualität, insbesondere charakterisiert durch eine Bromzahl ≦ 0,003, wird durch diese Verfahren jedoch nicht erreicht.

Es bestand daher die Aufgabe, ein Verfahren zur Herstellung von LAB's mit besonders hoher Reinheit, charakterisiert durch eine besonders niedrige Bromzahl, zu entwickeln. Die Aufgabe wird erfindungsgemäß durch die Kombination des Einsatzes von einem Olefinschnitt mit hohem Monoolefingehalt mit einem zweistufigen Alkylierungsverfahren gelöst, wobei in einer ersten Alkylierungsstufe durch die Umsetzung des Monoolefins hoher Konzentration mit Benzol ein LAB herkömmlicher Qualität entsteht und dieses LAB in einer nachgeschalteten kombinierten Alkylierung/Raffinationsstufe auf besonders einfache Weise mittels einer geeigneten Mischeinrichtung in ein LAB mit besonders niedriger Bromzahl und damit deutlich verbesserter Qualität überführt wird.

Gegenstand der Erfindung ist daher ein Verfahren zur Herstellung von linearen Alkylbenzolen mit besonders niedriger Bromzahl durch Alkylierung von Benzol mit Olefinen, dadurch gekennzeichnet, daß in einer ersten Stufe im Überschuß vorhandenes Benzol mit einem Olefinschnitt mit hohem Monoolefingehalt unter Verwendung von reinem Fluorwasserstoff als Katalysator zu linearen Alkylbenzolen alkyliert wird und daß in einer zweiten Stufe in einer Mischeinrichtung mit intensiver Durchmischung diese linearen Alkylbenzole nach deren destillativer Aufarbeitung nachfolgend mit Frischbenzol und redestilliertem Fluorwasserstoff raffiniert werden.

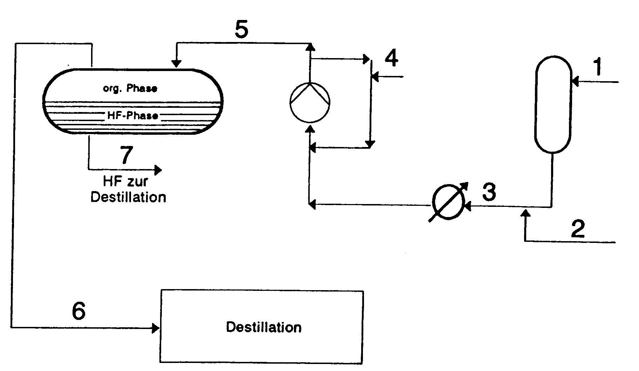

Das LAB verbesserter Qualität wird erhalten, in dem das LAB herkömmlicher Qualität (Stoffstrom 1, siehe Abb. 1) mit dem Stoffstrom 2 (Frischbenzol) vermischt und vorgewärmt wird und diese Mischung (Stoffstrom 3) zum Beispiel in einen durch Pumpen aufrechterhaltenen Mischkreislauf, der eine turbulente Durchmischung gewährleistet, eingespeist wird, dem der Katalysator HF (Stoffstrom 4) zugeführt wird. Der Stoffstrom 5 wird in den Absitzer gefördert, in dem sich der spezifisch schwerere Fluorwasserstoff als untere Phase (Stoffstrom 7) abscheidet. Die obere organische Phase (Stoffstrom 6) enthält überschüssiges Benzol, geringe Mengen HF und das LAB verbesserter Qualität. Diese Mischung wird destillativ so aufgearbeitet, daß das LAB verbesserter Qualität direkt als Sumpfprodukt abgenommen werden kann.

Es liegt sicher im allgemeinen nahe, eine Verbesserung der Produktqualität durch eine der Hauptreaktion folgende Nachreaktion durchzuführen. Wie das Vergleichsbeispiel 1 zeigt, reicht dieser zum Stand der Technik gehörende Raffinationsschritt jedoch nicht aus, die geforderte Produktqualität (hier insbesondere Bromzahl des LAB und Jodfarbzahl der Säure) zu erreichen.

Weiter liegt es im allgemeinen auch nahe, eine Verbesserung der Produktqualität durch Einsatz eines Ausgangsproduktes höherer Reinheit zu erreichen. Wie der Vergleichsversuch 2 zeigt, bringt jedoch der Einsatz von einem Olefinschnitt mit 96,5 % Monoolefin gegenüber dem Einsatz eines Paraffin/Olefin-Gemisches mit 27 % Olefin zwar eine gewisse Verbesserung, sie reicht jedoch ebenfalls nicht aus, die geforderten Reinheiten, charakterisiert durch die Bromzahl (≦ 0,003) und Jodfarbzahl der Säure (≦ 1), zu erfüllen.

Erst die erfindungsgemäße Kombination des Einsatzes eines Olefinschnittes mit Monoolefingehalten von mehr als 95 % mit der speziell gestalteten Raffinationsstufe führt zu linearen Alkylbenzolen besonders hoher Reinheit, mit den durch die im Beispiel angegebenen physikalischen Daten.

Dieses ist überraschend, da durch Weglassen des nach allgemeiner Ansicht als inertes Verdünnungsmittel dienenden Paraffins mit erhöhter volumenbezogener Umsatzgeschwindigkeit und damit problematischer Wärmeabfuhr mit wiederum dadurch bedingten thermisch verursachten Nebenreaktionen zu rechnen war.

Für die Vergleichsbeispiele und das erfindungsgemäße Beispiel wurden folgende Versuchsbedingungen gewählt:
Die Umsetzung in der ersten Stufe erfolgte bei beiden Vergleichsbeispielen und bei dem erfindungsgemäßen Beispiel bei 40 °C und einem Druck von 0,35 MPa. Das Verhältnis von Benzol zu Olefin betrug 6 : 1. Das Volumenverhältnis Fluorwasserstoffsäure zu Kohlenwasserstoffmischung betrug 1 : 1 (zweiphasige Mischung). Die Verweilzeit der ersten Stufe betrug 4 Minuten. Der Olefinschnitt hatte im Vergleichsbeispiel 1 einen Gehalt von 27 % und im Vergleichsbeispiel 2 und im erfindungsgemäßen Beispiel einen Gehalt von 96,5 % Monoolefin. Der Rest bestand beim Vergleichsbeispiel 2 und beim erfindungsgemäßen Beispiel aus 1,5 bis 2 % Paraffin und 1,5 bis 2 % Isoolefin.

In der zweiten Stufe, die beim Vergleichsbeispiel 1 und beim erfindinngsgemäßen Beispiel durchgeführt wurde, betrug die Temperatur während des Raffinationsschrittes 25 bis 30 °C. Das Verhältnis von Benzol/HF/Alkylbenzol betrug 1 : 6 : 10. Die Verweilzeit der zweiten Stufe betrug 1,5 Minuten.

Das Vergleichsbeispiel 2 wurde einstufig, d. h. ohne Raffinationsschritt durchgeführt.

| | Vergleichsbeispiel 1 | Vergleichsbeispiel 2 | Beispiel |
|---|---|---|---|
| Verfahren | zweistufig | einstufig | zweistufig |
| physikalische Daten | LAB ¹⁾ herkömmlicher Qualität | LAB ²⁾ herkömmlicher Qualität | LAB ²⁾ erfindungsgemäß |
| Farbe (APHA) gem. DIN 53409 | < 5 | < 5 | < 5 |
| Dialkyltetralin und -indangehalt [%] | 1,5 | 0,3 | 0,03 |
| Bromzahl nach Kaufmann [gBr₂/100 g LAB] | 0,010 | 0,008 | 0,002 |
| Jodfarbzahl der Säure gem. DIN 6162 | 2,5 | 1,3 | 0,55 |

| | | | |
|---|---|---|---|
| ¹⁾ = LAB hergestellt mit Paraffin/Olefin Gemisch mit einem Monoolefingehalt von 27 %, wobei das Olefin aus der Chlorierung/Dehydrochlorierung stammt | | | |
| ²⁾ = LAB hergestellt aus einem Olefinschnitt mit einem Monoolefingehalt von 96,5 % | | | |

## Patentansprüche

1. Verfahren zur Herstellung von linearen Alkylbenzolen mit besonders niedriger Bromzahl durch Alkylierung von Benzol mit Olefinen,
dadurch gekennzeichnet,
daS in einer ersten Stufe im Überschuß vorhandenes Benzol mit einem Olefinschnitt mit hohem Monoolefingehalt unter Verwendung von reinem Fluorwasserstoff als Katalysator zu linearen Alkylbenzolen alkyliert wird und daß in einer zweiten Stufe in einer Mischeinrichtung mit intensiver Durchmischung diese linearen Alkylbenzole nach deren destillativer Aufarbeitung nachfolgend mit Frischbenzol und redestilliertem Fluorwasserstoff raffiniert werden.

2. Verfahren nach Anspruch 1,
dadurch gekennzeichnet,
daß der Gehalt an Monoolefin im Olefinschnitt mindestens 95 % beträgt.

3. Verfahren nach Anspruch 1,
dadurch gekennzeichnet,
daß ein lineares Monolefin mit einer Kohlenstoffzahl von C₁₀-C₁₃ eingesetzt wird.

4. Verfahren nach Anspruch 1,
dadurch gekennzeichnet,
daß in der zweiten Stufe als Mischeinrichtung ein Kreislaufreaktor verwendet wird.

5. Verfahren nach Anspruch 1,
dadurch gekennzeichnet,
daß in der zweiten Stufe als Mischeinrichtung ein statischer Mischer verwendet wird.

6. Lineares Alkylbenzol mit besonders niedriger Bromzahl hergestellt nach den Ansprüchen 1 bis 5.
